# EUROPEAN PATENT SPECIFICATION

(11) **EP 2 007 212 B1**
(45) Date of publication and mention of the grant of the patent: **10.12.2014**
(21) Application number: 07736236.6
(22) Date of filing: 19.04.2007
(51) Int. Cl.: A23C 9/13, A23C 9/158, A23J 3/10, A23L 1/22

(54) **CASEIN MICELLES FOR NANOENCAPSULATION OF HYDROPHOBIC COMPOUNDS**
KASEINMIZELLEN FÜR DIE NANOVERKAPSELUNG VON HYDROPHOBEN VERBINDUNGEN
MICELLES DE CASÉINE POUR NANOENCAPSULATION DE COMPOSÉS HYDROPHOBES

(30) Priority: 20.04.2006 US 793302 P
(43) Date of publication of application: 31.12.2008
(73) Proprietor: Technion Research & Development Foundation Ltd., Haifa 32000 (IL)
(72) Inventor: LIVNEY, Yoav D., 20170 Misgav (IL); DALGLEISH, Douglas G., Guelph, Ontario N1C 1E9 (CA)
(74) Representative: Vossius & Partner
(86) International application number: PCT/IL2007/000496
(87) International publication number: WO 2007/122613

(56) References cited:
- WO-A-00/06108
- WO-A-02/064112
- WO-A-2004/000252
- GB-A- 2 041 378
- US-A- 5 173 322
- US-B1- 6 290 974
- FARRELL ET AL: "Casein micelle structure: What can be learned from milk synthesis and structural biology?" CURRENT OPINION IN COLLOID AND INTERFACE SCIENCE, LONDON, GB, vol. 11, no. 2-3, 8 February 2006 (2006-02-08), pages 135-147, XP005504281 ISSN: 1359-0294

## Description

### FIELD OF THE INVENTION

The present invention relates to the field of food technology and delivery of insoluble or hydrophobic biologically active compounds via beverages and food. In particular the present invention provides isolated casein micelles as defined by the claims useful for nanoencapsulation, stabilization and protection of hydrophobic active compounds and methods of producing same.

### BACKGROUND OF THE INVENTION

Casein, which accounts for about 80% of milk protein, is organized in micelles. Casein micelles (CM) are designed by nature to efficiently concentrate, stabilize and transport essential nutrients, mainly calcium and protein, for the neonate (*1*). All mammals' milk contains casein micelles. Cow's milk contains 30-35g of protein per liter, of which about 80% is within CM.

Micelles are spherical colloids, 50-500 nm in diameter (average of 150 nm) (*2*), made of the main four caseins: αₛ₁-casein (αₛ₁-CN), αₛ₂-CN, β-CN, and κ-CN (molar ratio ∼4:1:4:1 respectively) (*1-3*). The caseins are held together in the micelle by hydrophobic interactions and by bridging of calcium-phosphate nanoclusters bound to serine-phosphate residues present within the casein molecules (*1*).

The structure of the casein micelles is important for their biological activity in the mammary gland as well as for their stability during processing of milk into various products, as well as for the good digestibility of the nutrients comprising the micelles. The micelles are very stable to processing, retaining their basic structural characteristics through most of these processes (*1-3*).

However, it has not yet been possible to effectively harness these remarkable natural nanocapsules as nanovehicles for the delivery of added hydrophobic or poorly soluble biologically active compounds.

Caseinates have been used as microencapsulation wall materials (*4*). However, caseins forming such artificial capsules have lost the original micellar structure, as well as much of their natural functional behavior (e.g. during enzymatic coagulation of milk for cheese production). Moreover, the generally larger size of microcapsules is more likely to impair products smoothness.

CM can be re-assembled *in vitro,* by simulating their formation in the Golgi system of the mammary gland, according to a procedure developed by Knoop et al (*7*).

Vitamin D is a fat-soluble vitamin of great importance in calcium and phosphate metabolism, i.e. in facilitating of calcium absorption in the intestine, transporting calcium and phosphate to the bones, and re-absorption of calcium and phosphate in the kidneys. Vitamin D also takes part in the formation of osteoblasts, in fetal development and in the normal function of the nerve system, the pancreas and the immune system (*5*).

The recommended daily intake of vitamin D is 5 µg per day for adults between 21-51 years of age, and 10 µg per day for children and pregnant women. Fortified cereals, eggs, butter and fish oil are all vitamin D sources (*5*). However, vitamin D, being fat soluble is hardly found in skim milk and low-fat dairy products consumed in large quantities particularly in modem societies, being an important sources for calcium and phosphate.

Vitamin D has over 40 known metabolites, one of which is vitamin D2. Vitamin D2 originates from plants. It is found in nature in limited amounts, but can be synthesized readily, and therefore it is the main form of vitamin D used in the pharmaceutical industry. The vitamin structure contains double bonds that are sensitive to oxidation. Light, air and high temperature induce vitamin isomerization or degradation into inactive products (*5;6*).

Adsorption of hydrophobic nutraceuticals like vitamin D2, onto hydrophobic domains of the caseins, which tend to be found in the core of the micelle, would serve to stabilize these nutraceuticals in aqueous systems, protect them from degradation, and facilitate the enrichment of low fat and fat free dairy and other food products with these bioactive molecules, while minimizing the effect of their incorporation on the functional behavior of the system during processing.

Certain casein micelles are known in the art. US Patent No. 5,173,322 relates to the production of reformed casein micelles and to the use of such micelles as a complete or partial replacement for fat in food product formulations. Related US Patent No. 5,318,793 teaches powdered coffee whitener containing reformed casein micelles. US Patent No. 5,833,953 teaches a process for the preparation of fluoridated casein micelles, in which at least 100 ppm of a soluble fluoride salt are added to a solution comprising micellar casein.

US Patent No. 6,991,823 discloses a process for the preparation of mineral-fortified milk comprising the addition of an amount of a pyrophosphate or orthophosphate to the milk in order to enable the mineral to migrate into the protein micelles. It is to be explicitly understood that the present invention excludes mineral fortification of milk.

US Patent No. 6,290,974 teaches a food composition comprising a food additive comprising a preformed complex comprising β-lactoglobulin and a lipophilic nutrient selected from the group consisting of vitamin A, vitamin D, vitamin E, vitamin K₁, cholesterol, and conjugated linoleic acid. In that disclosure the lipophilic nutrient is bound to β-lactoglobulin via one or more amino acid residues, and in particular in proximity of or at the tryptophan 19 moiety of β-lactoglobulin.

US Patent No. 6,652,875 provides a formulation for the delivery of bioactive agents to biological surfaces comprising at least one isolated and purified casein protein or salt thereof in water. That invention relates to particular isolated and purified casein phosphoproteins in the form of casein calcium phosphate complexes intended to remain on the surface of oral cavity tissues or the gastrointestinal tract. There is neither teaching, nor suggestion regarding formation of nanoparticles, nor introduction of the bioactive compounds into nanoparticles.

US Patent Application Publication No. 2002/0054914 teaches a calcium phosphate/drug core with casein micelles reconstructed as aggregates around the cores, forming micellar structures, for the delivery of pharmaceutical agents. According to that disclosure, casein molecules are arranged, presumably as micelles, around calcium phosphate particles containing the active drug, and are linked to the therapeutic agent-containing microparticles by mainly calcium phosphate and electrostatic bond interactions. WO 02064112 discloses reformed casein micelles containing a therapeutic agent.

A paper by the inventor of the present invention published after the priority date of the present application describes introduction of exogenous hydrophobic biologically active compounds, including nutrients, nutraceuticals, drugs etc. into nano-sized casein micelles useful as carriers for such hydrophobic compounds, and in particular as a vehicle for the enrichment of a food or beverage product with a particular insoluble or poorly soluble agents (*8*). Such vehicles are not disclosed or suggested by the background art. Thus, there remains an unmet need in the food and beverage industry for compositions and methods useful in enhancing the nutritive value of a food or drink.

### SUMMARY OF THE INVENTION

The present invention is defined by the claims. The present invention provides casein micelles as defined by the claims as nanocapsular vehicles for hydrophobic biologically active compounds, particularly for nutraceuticals. The present invention departs from the known functions of casein micelles (CM) as a vehicle for minerals or their use as a fat substitute and utilizes reconstructed CM for encapsulation of insoluble or poorly soluble hydrophobic biologically active compounds. Molecules advantageously encapsulated within the CM include molecules having nutritional, therapeutic or cosmetic activity.

The present invention thus provides for the first time a system based on re-assembled casein micelles (rCM) for the delivery of hydrophobic biologically active compounds in food and beverages. According to certain embodiments, the food and/or beverages are low fat and non-fat. Advantageously, the system comprises only natural, generally regarded as safe (GRAS), non-toxic components.

The present invention provides a novel approach as defined by the claims for the nanoencapsulation and stabilization of hydrophobic biologically active compounds, particularly in non-fat or low fat edible products. The nano-capsules disclosed by the invention can be incorporated into a low-fat or non-fat dairy products or other food or beverage products without adversely modifying its properties. The system of the present invention is useful for encapsulation and delivery of sensitive health-promoting and cosmetic substances using natural, GRAS ingredients. It is to be explicitly understood that the present invention excludes mineral fortification of milk.

A major unique aspect of this invention is the harnessing of casein micelles for the stabilization, delivery and protection of insoluble and hydrophobic biologically active compounds, particularly nutraceuticals. The fully functional reconstituted CM not only are the ideal vehicles mimicking natural micelles to stabilize and deliver biologically active compounds, but their properties enable their incorporation into milk products without the need to modify any of the product properties or preparation process. Furthermore there is no need for an isolated and purified casein protein. For example, the rCM remain stable in acid or enzymatic curd formation during yogurt or cheese production. These benefits cannot be guaranteed using other casein-based preparations, which do not maintain the natural structure and properties of casein micelles.

The present invention is based in part on the surprising finding that vitamin D2 is between about 5 to about 22 fold more concentrated within re-constituted micelles than in the surrounding medium. Moreover, the morphology and average diameter of the re-assembled micelles were similar to those of naturally formed CM. Unexpectedly, the reconstructed or re-assembled CMs (rCM) also provide partial protection against light (e.g. UV-light)-induced degradation of a sensitive compound encapsulated within the CM, as exemplified herein for vitamin D2.

The present invention thus provides use of CM as defined by the claims as nanovehicles for entrapment, protection and delivery of sensitive hydrophobic biologically active compounds, including nutraceuticals, drugs and cosmetic products. Advantageously, the CM of the present invention enables delivery of the hydrophobic biologically active compound via low-fat or non-fat food and beverages, as well as in processed milk products. Thus, according to preferred embodiments, the nanovehicles of CM are used within low-fat and non-fat food and beverage products.

The present invention provides a re-assembled casein micelle as defined by the claims comprising at least one type of an exogenous hydrophobic or poorly watersoluble biologically active compound in the relatively more hydrophobic core of the micelle. In accordance with the invention the hydrophobic biologically active compound is selected from the group consisting of a drug, a nutraceutical, and a cosmetic compound.

In one embodiment the hydrophobic biologically active compound is selected from the group consisting of a peptide, a protein, an amino acid, a lipid, a proteoglycan, a polysaccharide, a vitamin, a hormone, a drug, a steroid a phytochemical, a flavorant, a sweetener, an anti-microbial, a preservative and the like.

According to certain currently preferred embodiments, the biologically active compound is a hydrophobic nutraceutical.

In some embodiments the nutraceutical is a fat-soluble vitamin. Suitable fat-soluble vitamins include vitamin D (D2, D3 and their derivatives), vitamin E (α, β, γ, δ-tocopherols, or α, β, γ, δ-tocotrienols), vitamin A (retinol, retinal, retinoic acid), vitamin K (K1, K2, K3 and their derivatives). In specific embodiments the vitamin is vitamin D.

In other embodiments the encapsulated compound is a sterol, cholesterol or its derivatives.

According to certain embodiments, the hydrophobic biologically active compound or nutraceutical is Coenzyme Q10.

In other embodiments, the biologically active compound or nutraceutical is selected from a carotenoid including α-, β-, or γ-carotene, lycopene, lutein, zeaxanthin, astaxanthin and others.

In yet other embodiments the nutraceutical is selected from an unsaturated fatty acid including linoleic acid, conjugated linoleic acid, linolenic acid, omega-3 fatty acids including but not limited to docosahexaenoic acid (DHA) and eicosapentaenoic acid (EPA) and their glycerol-esters.

In some embodiments the nutraceutical is selected from phytoestrogens including phytosterols (e.g. β-sitosterol, campesterol, stigmasterol etc.), isoflavones (genistein, daidzein), stilbenes (e.g. trans-resveratrol), lignans (e.g. Matairesinol) and coumestans (e.g. coumestrol), and others.

In yet other embodiments the nutraceutical is selected from a bioactive peptide, such as casein-phosphopeptide (CPP) and other calcium-binding peptides.

The casein micelle may be about 50nm to about 500nm in diameter. The average diameter of the casein micelles may also be about 100-150nm.

In another aspect the present invention provides a composition comprising a re-assembled casein micelle as defined by the claims, the micelle comprising a hydrophobic biologically active compound within the relatively hydrophobic core of the micelle.

In some embodiments the composition is selected from a non-fat or low fat food product or beverage.

In certain embodiments, the biologically active compound is selected from the group consisting of nutraceuticals, drugs and cosmetic products. According to certain currently preferred embodiments, the biologically active compound is a nutraceutical.

In yet another aspect the present invention provides a method for the preparation of a re-assembled casein micelle, the micelle comprising at least one hydrophobic or poorly water soluble biologically active compound within the micelle, the method comprising the steps of:
a) preparing an aqueous solution comprising a source of casein;
b) adding a cosolvent solution comprising at least one type of hydrophobic biologically active compound to the casein solution;
c) adding a source of citrate ions, a source of phosphate ions and a source of calcium ions to the mixture of step (b) to form a nano-sized micelle dispersion;
d) adjusting the pH of the dispersion to stabilize the nano-sized micelles.

According to certain embodiments, step (c) of the method comprises
(i) adding the source of phosphate ions and the source of citrate ions to the mixture of step (b);
(ii) preparing a solution comprising calcium ions as the source of calcium ions; and
(iii) combining the mixture of step (i) with the calcium solution of step (ii) using high pressure homogenization.

In some embodiments the method further comprises the step of drying the micelle dispersion.

The cosolvent used to prepare the solution comprising at least one hydrophobic compound can be any food grade water miscible organic solvent, which evaporates during the drying of the micelles. Natural or synthetic solvents as are known in the art can be used according to the teaching of the present invention. In some embodiments the solvent is ethanol.

The source of casein may be sodium caseinate. The source of casein may also be milk, or milk powder, or any soluble caseinate or casein preparation, or isolated alpha, beta, and/or kappa casein or mixtures of such caseins.

The source of citrate ions may be provided as tri-potassium citrate, or tri-sodium citrate or any food-grade citrate salt, preferably a source of citrate that is derived from milk or any other natural food source.

The source of phosphate ions may be provided as K₂HPO₄ or Na₂HPO₄ or any food-grade phosphate salt. The phosphate source may derive from milk or any other natural food source.

The source of calcium ions may be provided as CaCl₂, CaF₂, or calcium citrate, or any food-grade calcium salt, preferably calcium salt derived from milk or any other natural food source.

In some embodiments the solution comprising casein comprises about 1% to about 20% caseinate, typically 3-8%, more typically 4%-6% casein in an aqueous solution. The aqueous solution may also contain a food grade organic solvent

In some embodiments the pH is adjusted to a pH in the range of about 6 to about 7.5, preferably in the range of about pH 6.5 to about 7.0.

In an optional embodiment the dispersion of step d) is dried to produce a water-dispersible dry product. Suitable methods of drying a dispersion include freeze-drying, spray-drying, drum-drying or any other method known to one with skill in the art.

### BRIEF DESCRIPTION OF THE FIGURES

**Figures 1A-1B** presents RP-HPLC chromatograms of vitamin D2 in (A) centrifugation-pellets of rCM and of D2-rCM, and in (B) supernatants of rCM and of D2-rCM
**Figures 2A-2C** shows size distributions of (A) rCM, (B) D2-rCM and (C) naturally occurring CM in skim milk.
**Figures 3A-3C** presents Cryo-TEM images of (A) rCM, (B) D2-rCM and (C) naturally occurring CM in skim milk. The Bar on the bottom right is 100 nm long. (The dark area on the bottom is the perforated carbon film holding the sample.)
**Figure 4** shows the size distribution of vitamin D-containing CM prepared by ultra-high pressure homogenization immediately following in-line blending of vitamin D-containing caseinate solution and a calcium solution.
**Figure 5** presents absorbance spectra of 2.5% caseinate and 31.5µM Vitamin D2 solutions in the range of 210-360 nm. Dashed line Caseinate; Solid line- Vitamin D2.
The vertical line at 254 nm marks the wavelength of the UV lamp used during the exposure experiments.
**Figure 6** shows a cryo-TEM image of CM encapsulating vitamin D₂, reassembled using ultra high pressure homogenization.

### DETAILED DESCRIPTION OF THE INVENTION

The present invention is defined by the claims. The present invention provides isolated casein micelles and methods as defined by the claims for encapsulation of hydrophobic or poorly water-soluble biologically active compounds in CM, with minimal changes to the functional properties of both the micelles and the active compound.

The present invention now discloses that absorption of hydrophobic biologically active compounds, for example nutraceuticals, onto the hydrophobic domains of caseins, and the reformation of the micelles stabilize the hydrophobic compound in aqueous surrounding and protect them from degradation. Such casein micelles-hydrophobic compound system facilitates the enrichment of low fat and fat free dairy and other food products with the bioactive molecules, while minimizing the effect of the compound incorporation on the food properties in general and during processing. Encapsulation of biologically active compounds within casein micelles is advantageous over hitherto known encapsulation methods as the micelles are a natural component of milk products and their nanometric size minimizes their effect on the food product, including dairy as well as non dairy foods. In addition, when the active compound possesses undesirable attributes, the encapsulation in the micelles diminishes such unwanted features (e.g. in the case of omega 3 fatty acids). Another important potential benefit is the improved bioavailability of the enclosed compound due to its distribution, at a molecular level, over a very large surface area of the caseins in the nanoscopic micelles, and the fact that caseins are evolutionally optimized for ease of digestion and absorption. The open tertiary molecular structure of casein also facilitates effective proteolysis.

Described herein is a method for incorporation of vitamin D2 into CM, and evaluation of the encapsulation process by: (a) evaluation of the efficiency of encapsulation, i.e. the percent of added vitamin D2 which was incorporated into the micelles, (b) preservation of micelle properties: diameter as determined by dynamic light scattering (DLS) and morphology (as determined by cryo-TEM), (c) evaluation of the protective effect of the micelles over vitamin D2 from photochemical degradation induced by UV exposure.

### Definitions

For convenience and clarity certain terms employed in the specification, examples and claims are described herein.

As used herein, the term "casein" refers to the predominant protein in non-human mammals and human milk, comprising the subgroups α_{S1}, α_{S2}, β and κ.

The term "biologically active compound" encompasses a compound having a therapeutic, nutritional and/or cosmetic activity. Biologically active compounds according to the teaching of the invention include, but are not limited to peptides, proteins, amino acids, lipids, proteoglycans, polysaccharides, vitamins, hormones, drugs, steroids, phytochemicals, polynucleotides, flavorants, sweeteners, an anti-microbials, and preservatives.

A "nutraceutical", also known as a functional food (or its component), is generally any one of a class of dietary supplements, vitamins, minerals, herbs, healing or disease-preventative foods that have medical or pharmaceutical effects on the body. Exemplary non-polar or hydrophobic nutraceuticals include, but are not limited to fatty acids (e.g., omega-3 fatty acids, DHA and EPA); fruit and vegetable extracts; vitamins A, D, E and K; phospholipids, e.g. phosphatidyl-serine; certain proteoglycans such as chondroitin; certain amino acids (e.g., iso-leucine, leucine, methionine, phenylanine, tryptophan, and valine); various food additives, various phytonutrients, for example lycopene, lutein and zeaxanthin; certain antioxidants; plant oils; and fish and marine animal oils and algae oils. It is to be understood that certain nutraceuticals can be also referred to as therapeutics as well as cosmetic compounds.

The following examples are presented in order to more fully illustrate some embodiments of the invention. They should, in no way be construed, however, as limiting the broad scope of the invention. One skilled in the art can readily devise many variations and modifications of the principles disclosed herein without departing from the scope of the invention.

### EXAMPLES

### Materials

Sodium caseinate (Miprodan 30, 93.5% protein, MD foods ingredients amba, Videbaek, Denmark). Vitamin D2 (Sigma-Aldrich, Rehovot, Israel). Ethanol (absolute), hydrochloric acid (concentrated), (Bio-Lab, Jerusalem, Israel). Tripotassium citrate, sodium hydroxide, (Merck, Darmstadt, Germany). Calcium chloride (Carlo Erba, Rodano, Italy). Dipotassium hydrogen phosphate (Spectrum, CA, USA).

Materials for analysis only: petroleum ether and diethyl ether (Bio-Lab, Jerusalem, Israel). Potassium hydroxide and pyrogallol (Merck, Darmstadt, Germany). Ethylene diamine tetraacetic acid (EDTA) (Acros, NJ, USA). Methanol and acetonitrile (both HPLC grade) (Lab Scan, Dublin, Ireland).

### Methods

***Non covalent binding of vitamin D2 to sodium caseinate*** was achieved by dropwise addition of 1-100mM, typically 2-50mM, more typically 5-20mM, more typically 15 mM solution of the vitamin in absolute ethanol into a 1-20% caseinate, typically 3-8%, more typically 5% caseinate solution, while stirring, to a final concentration of about 5-50,000 µM, typically about 10-5000 µM, more typically about 25-1000, more typically 25-100, more typically about 65 µM.
***Re-assembly** of **CM.*** Preparation of re-assembled CM (rCM) was done based on the method described by Knoop et al. (8). However, unlike Knoop et al. the casein is provided as a rehydrated commercial sodium caseinate powder, rather than a freshly prepared caseinate, in order to extend the commercial applicability of the method. To 2-10%, more typically 5% non-enriched caseinate solution and to vitamin D2 enriched caseinate solution (200 mL each), 0.5-1.5M, more typically 1 M tri-potassium citrate (4 mL), 0.05-0.5M, more typically 0.2 M K₂HPO₄ (24 mL) and 0.05-0.5M, more typically 0.2 M CaCl₂ (20 mL) were added. Eight consecutive additions of the K₂HPO₄ solution (2.5 mL) and of the CaCl₂ (5 mL) were performed, at 15-minute intervals. During this process, samples were stirred in a thermostated bath at 25-42°C, more typically 37°C. The pH was maintained between 6.5-7.0, more typically between 6.7-7.0, using 0.1N HCl or 1N NaOH. The volume was then adjusted to 400 mL with water, the pH was corrected to 6.5-7.0, more typically to 6.7, and the final dispersions were stirred moderately for one hour (*8;9*).

Alternatively, phosphate and optionally also citrate are added to the aqueous casein solution containing the hydrophobic molecule and the mixture is blended to make solution A. For example, this solution contains 400mL 5% sodium caseinate, 2mL 5mg/mL vitamin D2 in absolute ethanol, 88mL 0.4M K2HPO4 and 10mL 0.8M sodium citrate. A calcium ion source solution (B) is prepared separately. The calcium solution comprises, for example, 300 mL 0.08M CaCl2. Solutions A and B are then combined during a high pressure homogenization process. Ultra-high pressure homogenization may be done by any method known in the art. For example, ultra-high pressure homogenization is performed using a Micro DeBee ultra high pressure homogenizer, at 20-25kPSI, using a 0.1 mm orifice and 1 mm reactor cylinders, without back pressure, at a temperature of ∼40°C. Flow rates are adjusted so that the main stream (solution A) and the added stream (B) are processed simultaneously throughout the process. *Analytical fractionation:* Micelle preparations were centrifuged at 20 °C and 25,000xg for one hour and the supernatant was separated from the pellet by decantation. The supernatant was then ultra-filtered using Amicon 8050 stirred ultrafiltration cell with a 10,000 Da nominal molecular weight limit membrane (Millipore). All fractions were collected and analyzed for vitamin D2 content.

### Evaluation of micelle protection against UV light induced degradation of Vitamin D2:

Samples containing vitamin D2-enriched rCM (D2-rCM) were placed in a wooden light-proof cabinet, and exposed to a 254 nm UV light, at 200 µW/cm² intensity for 3, 6, 12, and 24 hours. At each exposure time, three 20 mL samples were compared: a micelle dispersion preparation, a negative control (an identical sample covered with an aluminum foil to completely block the UV), and another control containing only serum from the D2-rCM preparation, which was exposed to UV. The serum samples were obtained by centrifuging D2-rCM dispersion and collecting the supernatant.

***UV spectra determination for caseinate and vitamin D2***. Samples of caseinate and vitamin D2 at concentrations similar to their concentrations in the rCM suspension (2.5% and 31.75 µM respectively) were prepared. UV absorbance spectra of the samples were analyzed by absorbance scan at wavelengths between 220 nm and 360 nm, using a Pharmacia Biotech Ultrospec 3000 spectrophotometer.

***Saponification and extraction:*** Pellets were resuspended in a 100 mM EDTA solution of same weight as the removed supernatant, and equilibrated for 6 hours at 4°C. Both pellet, serum and permeate from each sample underwent saponification and extraction procedures based on Renken and Warthesen (*10*): Five mL of each sample were placed into a 25 mL glass stoppered round bottom flask wrapped with aluminum-foil. 3 mL of 5% KOH and 1.5 mL of 1% ethanol pyrogallol solutions were added. The samples were flushed with nitrogen, capped and then left to stir slowly in the dark for 12 hours at room temperature.

Each sample was then poured into a separatory funnel. The round bottomed flask was washed with 2 mL of water, then 0.75mL of ethanol, and lastly 5mL of petroleum ether: diethyl ether (90:10 v/v), adding each wash liquid into the separatory funnel. The mixture was gently mixed and the phases were allowed to separate. The hydrophilic phase was then poured into a second separatory funnel adding 0.75 mL ethanol and 5 mL of the ether mixture. After gentle mixing the phases were allowed to separate. The hydrophobic phase was put into the first separatory funnel. 4.5 mL water was used to wash the hydrophobic phase four times (*10*). The hydrophobic phase was collected and the solvents were evaporated using nitrogen. The dried sample was re-suspended in 1 mL solution of methanol:water (93:7 v/v) (*11*).

***Determination of vitamin** D2 **content:*** Vitamin D2 analysis was done by reverse phase HPLC (RP-HPLC). All samples were analyzed for vitamin D2 using a 4.6x100 mm C18-C2 RP-HPLC column and a UV detector at 265 nm. The gradient used was zero to 75% acetonitrile as eluent B, while methanol:water (93:7 v/v) serve as eluent A (*11*). Calibration curve was prepared using vitamin D2 standard in methanol:water (93:7 v/v) solution at 7 concentrations ranging from 5 to 250 µg/mL.

Vitamin D2 fractions were collected during RP-HPLC and analyzed for UV absorbance spectrum from 220 nm to 360 nm for further identity validation, using a Pharmacia Biotech Ultrospec 3000 spectrophotometer.

***Size and morphology determination of rCM*:** For both rCM and D2-rCM, average size was measured by dynamic light scattering (DLS) (BIC 90Plus, Brookhaven Instruments Corp.). Morphology was determined using Cryogenic Transmission Electron Microscopy (Cryo-TEM): Specimens were prepared in a controlled environment vitrification system (CEVS) at controlled temperature and humidity to avoid loss of volatiles. The samples were brought to a desired temperature (25 °C and 35 °C) and allowed to equilibrate in the CEVS for an hour. Then, a 7 µl drop of the examined dispersion was placed on a TEM copper grid covered with a perforated carbon film, and blotted with a filter paper to form a thin liquid film of the sample (100-200 nm thick). The thinned sample was immediately plunged into liquid ethane at its freezing temperature (-183 °C) to form a vitrified specimen, and then transferred to liquid nitrogen (-196 °C) for storage until examination. The vitrified specimens were examined in a Philips CM120 transmission electron microscope operating at an accelerating voltage of 120 kV. An Oxford CT3500 cryo-specimen holder was used to maintain the vitrified specimens below -175 °C during sample transfer and observation. Specimens were recorded digitally on a cooled Gatan MultiScan 791 CCD camera using the Digital Micrograph 3.1 software, in the low-dose imaging mode to minimize beam exposure and electron-beam radiation damage.

Brightness and contrast adjustments were done using Photoshop 7.0 ME.

***Durability of the** micelles to **high shear*** Samples of rCM and D2-rCM suspensions, as well as a sample of skim milk reconstituted from powder, were homogenized using a Micro DeBee ultra high pressure homogenizer, by 1 pass at the single-reversed-flow mode at 185 ± 10 MPa, using a 0.1mm orifice, and a back-pressure of 10 ± 3 MPa. Average diameter of rCM and D2-rCM was measured before and after homogenization process by DLS (see method details above). Relative average diameter changes were then determined for each sample.

### Analyses

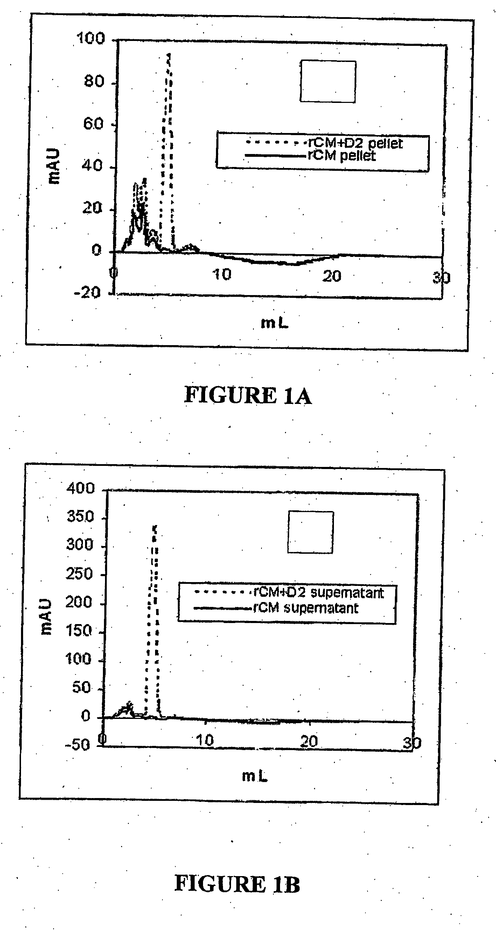

Figure 1 presents the results of the analysis of vitamin D2 in preparations of micelles enriched with vitamin D2 (D2-rCM) and control rCM preparations without the vitamin. Both analyses of the micelle pellets obtained by centrifugation and of their respective serum fractions are presented. In the chromatograms of the control rCM preparation fractions (pellet - Fig1A, and serum - Fig 1B) vitamin D2 peaks were absent, while in both D2-rCM fractions those peaks were observed. UV absorbance spectra of the peaks identified as vitamin D2 indicated good matching between vitamin D2 standard and vitamin D2 eluted at the same position in the sample runs.

During the analysis, 45-95%, more typically 65-85% of total vitamin D2 added were recovered by the extraction procedure from the serum and the pellet together. 25-75%, more typically 45-60% of the recovered vitamin D2 were found to be incorporated into the micelles, which accounted for 2-15%, more typically 8% by weight of the total D2-rCM suspension prepared.

It was determined that vitamin D2 concentration in the rCM was about 2-22 times, more typically 5-10 times greater than its concentration in serum: 44-57 µg/mL vs. 2-8 µg/mL respectively. Therefore, fortification of milk using such vitamin D2-enriched rCM accounting for only 0.001-1%, more typically 0.1-1%, more typically 0.5-0.6% of the total milk casein, would provide about one third of the vitamin D2 recommended daily allowance (RDA) for adults in a single glass of milk (200mL).

### Size and morphology determination of rCM

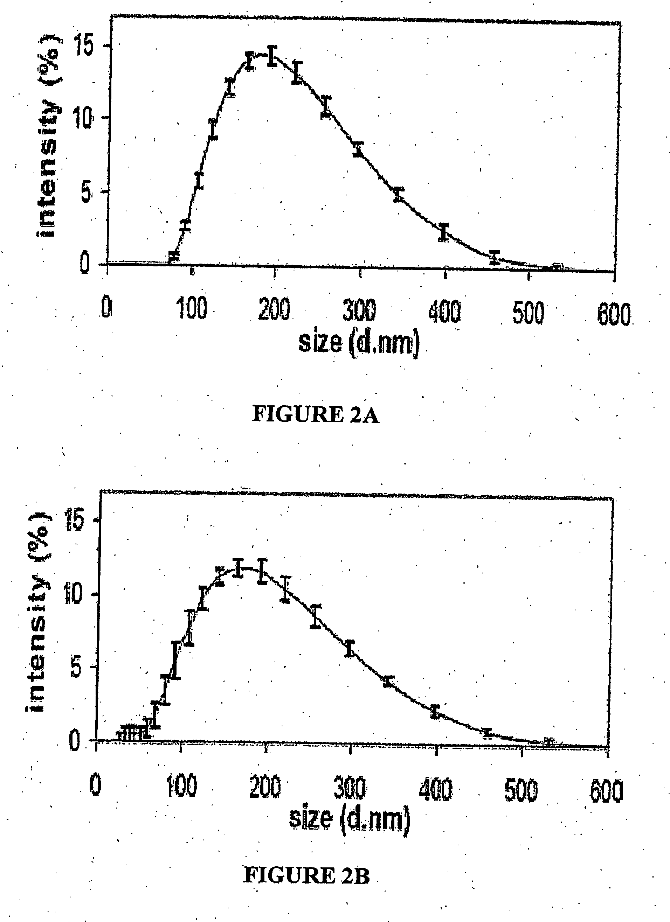

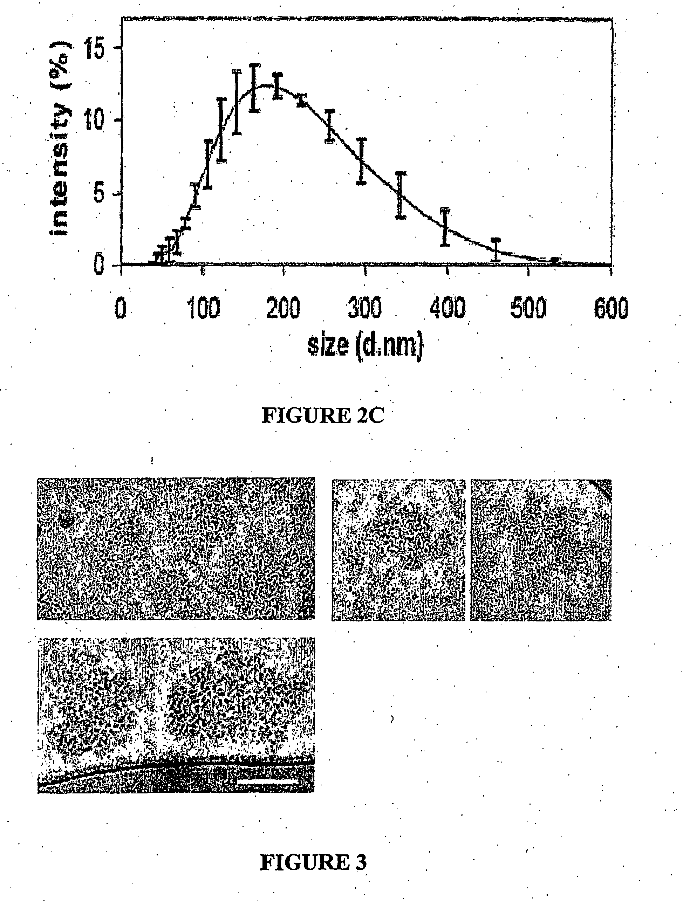

The re-assembled micelles had average diameters of 146 and 152 nm without and with vitamin D2 respectively (Figure 2). As mentioned hereinabove, the normal size range of CM in milk is ∼50-500 nm, and the average is ∼150 nm.

D2-rCM and rCM had similar morphology, which was also typical to naturally occurring CM, as may be judged by the available resolution of the TEM micrographs (Figure 3). These micrographs suggest that the incorporation of vitamin D2 has minimal effect over the morphology of the CM.

### Shear stability of rCM and D2-rCM

Following an ultra-high-pressure homogenization process the average diameter of rCM was reduced to 122 nm (26% reduction) and that of D2-rCM was reduced to 125 nm (27% reduction). The reference micelles from reconstituted skim milk showed a 9% reduction in diameter during the homogenization. While this shows that the reformed micelles are expectedly somewhat weaker than the original micelles, their durability through such extreme shear suggests they could well withstand typical processing shear which is seldom that high. The similar extent of reduction in size for rCM and D2-rCM suggests that the incorporation of vitamin D2 into rCM did not weaken their structure as reflected by shear stability.

### Ultra-high pressure homogenization used for preparation of rCM

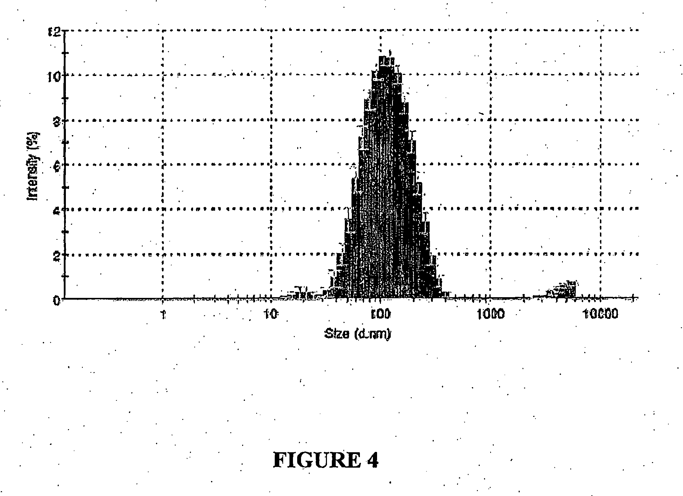

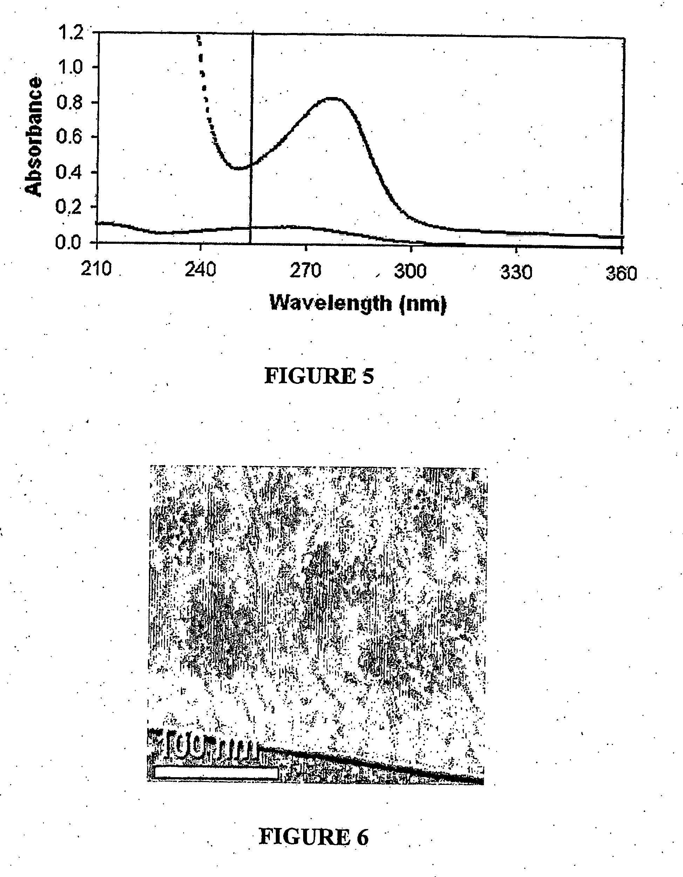

Figure 4 shows the size distribution of CM obtained by in-line merging of two streams, just before the high pressure chamber of a high-pressure homogenizer. One stream was the aqueous casein solution containing the hydrophobic molecule(s), as well as phosphate and optionally also citrate, and the other stream was a calcium ion source solution. The average size of the CM obtained was ∼100nm. Figure 6 shows a cryo-TEM image of the vitamin D2-containing CM obtained this way. Analysis of vitamin D2 in the micelle-pellet and supernatant of the centrifuged micelle preparation showed 4-5 times higher vitamin concentration in the micelle pellet compared to the supernatant (serum). The pellet contained 30.3 ± 0.4 and the supernatant contained 6.7 ± 0.7 micrograms/mL of the vitamin.

### Quantification of the protective effect of the micelles against UV light-induced photochemical degradation of vitamin D2

Table 1 presents vitamin D2 degradation as the remaining percent of the initial concentration in each fraction with exposure time, in D2-rCM suspension exposed to UV light, D2-rCM suspension unexposed to UV light (control I) and in D2-rCM suspension serum exposed to UV light (control II). (UD=undetectable).

**Table 1: Percent of vitamin D2 remaining in micelle preparation following UV exposure**

| Exposure Time (Hrs). | UV exposed micelle suspension | | Unexposed micelle suspension (control I) | | UV exposed serum (control II) |
|---|---|---|---|---|---|
| | Micelle pellet | Serum | Micelle pellet | Serum | |
| 0 | 100 | 100 | 100 | 100 | 100 |
| 3 | 20.9 | 7.2 | 107.8 | 43.1 | 0.11 |
| 6 | 2.7 | 1.7 | 41.0 | 74.9 | UD |
| 12 | 2.7 | 0.5 | 78.5 | 77.1 | UD |
| 24 | 1.8 | 0.9 | 135.1 | 62.0 | UD |

The data in Table 1 merits several observations: First, the comparison of the UV exposed serum (control II) to the serum of control I (unexposed) shows how relatively quickly photochemical degradation of unprotected vitamin D2 occurs. The vitamin in the serum is presumably bound to residual soluble casein molecules which did not aggregate into micelles. The main interesting observation is the comparison of the rate of degradation of the vitamin within the micelles in the exposed preparation to that of the UV exposed serum (control II). This comparison demonstrates the significant relative protection conferred by the micelles to the encapsulated vitamin. The micelles also confer some protection to the vitamin in their surrounding serum, as the rate of degradation in the serum of the exposed micelle dispersion was lower than that in the exposed micelle-free serum (control II). This may be explained by a "shade" effect of the micelles which block and absorb much of the light. Lastly, it is observed that the degradation of the vitamin in the micelles of the unexposed preparation of control I (although slightly obscured by experimental error) was slower than in the serum of this preparation. This degradation may be due to chemical oxidation, (e.g. by dissolved oxygen) and this observation suggests that the micelles confer some protection against chemical degradation as well, however this remains to be verified by other experiments.

The nature of the protective effect of the micelles against photo-degradation of the vitamin was examined by comparing the absorbance spectra for both caseinate and vitamin D2 components in the rCM suspension

As is shown in Figure 5, at the concentrations examined for each of the fractions (caseinate and vitamin D2) in the rCM suspension, caseinate, being a protein with aromatic side groups and double-bonds, absorbs significantly more UV light than vitamin D2. These data support the conclusion that casein micelles have protective effect for the vitamin D2 enclosed therein and to some degree also to vitamin D2 around the micelles.

Casein micelles were shown to be potential nano-vehicles for added nutraceuticals such as the fat-soluble vitamin D2 chosen here as a model. In terms of encapsulation efficiency, about 25% to about 75%, more typically 45-60% of the vitamin retrieved from the micelle suspension was found in the reformed micelles - which contained about 2 to about 22 fold, more typically 4-10 fold higher concentration of the vitamin compared to the surrounding medium. Some vitamin D may be lost by binding to hydrophobic domains of unaggregated proteins in the serum. The extraction-based analysis method allowed the retrieval of about 45 to about 95%, more typically 65-85% of the added vitamin. The micelles' morphology and size were similar to those of naturally occurring CM, in accord with the purpose to minimize modification of micelle properties. It was also shown that apart from their effectiveness in stabilizing oil-soluble compounds in aqueous environment, the rCM have an additional protective affect against photochemical degradation of the entrapped hydrophobic compound, for example the nutraceutical vitamin D2.

### REFERENCES

1. DeKruif, C.G.; Holt, C. Casein micelle structure, function and interactions. In Advanced Dairy Chemistry-1 Proteins Part A; 3 Fox, P F; McSweeney, P.L.H., Eds.; Kluwer Academic/ Plenum Publishers: New York, 2005; 233-276.
2. Fox, P.F. Milk proteins: general and historical aspects. In Advanced Dairy Chemistry-1 Proteins Part A; 3 Fox, P F; McSweeney, P.L.H., Eds.; Kluwer Academic/ Plenum Publishers: New York, 2005; 1-48.
3. Swaisgood, H.E. Chemistry of the caseins. In Advanced Dairy Chemistry-1 Proteins Part A; 3 Fox, P F; McSweeney, P.L.H., Eds.; Kluwer Academic/ Plenum Publishers: New York, 2003; 139-202.
4. Hogan, S.A.; McNamee, B.F.; O'Riordan, E.D.; O'Sullivan, M. Microencapsulating Properties of Sodium Caseinate. Journal of Agricultural and Food Chemistry 2001, 49, 1934-1938.
5. Eitenmiller, R.R.; Landen, W.O., Jr. Vitamin D. In Vitamin analysis for the health and food science; CRC Press: Boca Raton, 1999; 77-82.
6. Bell, A.B. The chemistry of the vitamins D. In Vitamin D; Lawson, D E M, Ed.; Academic Press: London, 2005; 1-41.
7. Knoop, A.M.; Knoop, E.; Wiechen, A. Sub-structure of synthetic casein micelles. Journal of Dairy Research 1979; 46, 347-350.
8. Semo, E.; Kesselman, E.; Danino, D.; Livney, Y.D. Casein micelle as a natural nano-capsular vehicle for nutraceuticals. Food Hydrocolloids 2007; 21, 936-942.
9. Aoki, T.; Tanaka, H.; Kako, Y. Incorporation of individual casein constituents into micelles in artificial casein micelles. Nippon Chikusan Gakkaiho 1989; 60, 583-589.
10. Renken, S.A.; Warthesen, J.J. Vitamin D stability in milk. Journal of Food Science 1993; 58, 552-6, 566.
11. Mattila, P.; Konko, K.; Eurola, M.; Pihlava, J.M.; Astola, J.; Vahteristo, L.; Hietaniemi, V.; Kumpulainen, J.; Valtonen, M.; Piironen, V. Contents of vitamins, mineral elements, and some phenolic compounds in cultivated mushrooms. Journal of agricultural and food chemistry 2001; 49, 2343-2348.

## Claims

1. A re-assembled casein micelle entrapping at least one exogenous hydrophobic biologically active compound within the micelle, wherein the hydrophobic biologically active compound is adsorbed onto the hydrophobic domains of caseins and is selected from the group consisting of a drug, a nutraceutical, and a cosmetic compound.

2. The micelle according to claim 1, wherein the re-assembled casein micelles have an average diameter of 50 nm to 150 nm.

3. The micelle according to claim 1 or 2,
wherein the hydrophobic biologically active compound is selected from the group consisting of a peptide, a protein, amino acid, a lipid, a proteoglycan, a polysaccharide, a vitamin, a hormone, a drug, a steroid, a phytochemical, a polynucleotide, a flavorant, a sweetener, an anti-microbial and a preservative; or wherein the hydrophobic biologically active compound is a fat-soluble vitamin selected from vitamin A, vitamin E, vitamin D and vitamin K, and derivatives thereof; or
wherein the vitamin is vitamin D; or
wherein the hydrophobic biologically active compound is selected from the group consisting of sterol cholesterol and derivatives thereof; or
wherein the hydrophobic biologically active compound is selected from α-carotene, β-carotene, γ-carotene, lycopene, lutein, zeaxanthin, and astaxanthin; or
wherein the hydrophobic biologically active compound is an unsaturated fatty acid, preferably wherein the unsaturated fatty acid is selected from the group consisting of linoleic acid, conjugated linoleic acid, linolenic acid, omega-3 fatty acids, induding DHA and EPA and their glycerol-esters; or
wherein the hydrophobic biologically active compound is selected from a phytoestrogen, a phytosterol, an isoflavone, a stilbene, a lignan and a coumestan; or
wherein the hydrophobic biologically active compound is Coenzyme Q10.

4. A composition comprising a re-assembled casein micelle according to any one of claims 1 to 3.

5. The composition according to claim 4, wherein the hydrophobic biologically active compound is a nutraceutical.

6. The composition according to claim 4, wherein the composition is selected from a low fat and non-fat dairy product.

7. The composition according to claim 6, wherein the composition is skim milk.

8. The composition according to claim 6, wherein the composition is low fat or non-fat yogurt.

9. A method for the preparation of a re-assembled casein micelle, the micelle entrapping at least one hydrophobic biologically active compound within the micelle wherein the biologically active compound is selected from the group consisting of a nutraceutical, a drug and a cosmetic product, the method comprising the steps of:
a) preparing an aqueous solution comprising a source of casein;
b) adding a cosolvent solution comprising at least one type of hydrophobic biologically active compound to the casein solution;
c) adding a source of citrate ions, a source of phosphate ions and a source of calcium ions to the mixture of step (b) to form a nano-sized micelle dispersion; and
d) adjusting the pH of the dispersion to stabilize the nano-sized micelles.

10. The method according to claim 9, wherein step (c) comprises:
(i) adding the source of phosphate ions and the source of citrate ions to the mixture of step (b);
(ii) preparing a solution comprising calcium ions as the source of calcium ions; and
(iii) combining the mixture of step (i) with the calcium solution of step (ii) using a high pressure homogenization.

11. The method according to claim 9 or 10, further comprising the step of drying the micelle dispersion; or wherein the solution comprises about 1% to about 20% casein; or wherein the pH is adjusted to a pH in the range of about 6.0 to about 7.5.

12. The method of claim 9, wherein the cosolvent is a food grade water miscible organic solvent.

13. The method of claim 12, wherein the cosolvent is ethanol.

14. The method of claim 9, wherein the re-assembled casein micelles have an average diameter of 50nm to 100nm.

15. Use of a re-assembled casein micelle compound according to claim 1 or 9 as nanovehicle for the entrapment, protection and delivery of sensitive hydrophobic biologically active compounds, wherein the sensitive hydrophobic biologically active compounds are adsorbed onto the hydrophobic domains of caseins.

16. The use of claim 15, wherein the casein micelle entrapping the sensitive hydrophobic biologically active compounds has an average diameter of 50 nm to 150 nm.

17. The use of claim 15 or 16, wherein the sensitive hydrophobic biologically active compounds are nutraceuticals, drugs or cosmetic products.

## Patentansprüche

1. Wieder-zusammengesetzte Kasein-Mizelle, die mindestens eine exogene hydrophobe biologisch aktive Verbindung in der Mizelle einschließt, wobei die hydrophobe biologisch aktive Verbindung an die hydrophoben Domänen der Kaseine adsorbiert ist und ein Medikament, ein Nutraceutical oder eine kosmetische Verbindung ist.

2. Mizelle nach Anspruch 1, wobei die wieder-zusammengesetzten Kasein-Mizellen einen durchschnittlichen Durchmesser von 50nm bis 150nm haben.

3. Mizelle nach Anspruch 1 oder 2,
wobei die hydrophobe biologisch aktive Verbindung ein Peptid, ein Protein, eine Aminosäure, ein Lipid, ein Proteoglykan, ein Polysaccharid, ein Vitamin, ein Hormon, ein Medikament, ein Steroid, ein sekundärer Pflanzenstoff, ein Polynukleotid, ein Geschmacksstoff, ein Süßstoff, eine antimikrobielle Substanz oder ein Konservierungsstoff ist oder
wobei die hydrophobe biologisch aktive Verbindung ein fettlösliches Vitamin ist, ausgewählt aus Vitamin A, Vitamin E, Vitamin D und Vitamin K und Derivaten davon oder
wobei das Vitamin Vitamin D ist oder
wobei die hydrophobe biologisch aktive Verbindung Sterolcholesterin oder ein Derivat davon ist oder
wobei die hydrophobe biologisch aktive Verbindung α-Carotin, β-Carotin, γ-Carotin, Lycopen, Lutein, Zeaxanthin oder Astaxanthin ist oder
wobei die hydrophobe biologisch aktive Verbindung eine ungesättigte Fettsäure ist, wobei die ungesättigte Fettsäure bevorzugt ausgewählt ist aus der Gruppe bestehend aus Linolsäure, konjugierter Linolsäure, Linolensäure, Omega-3-Fettsäuren, einschließlich DHA und EPA und deren Glykolester oder
wobei die hydrophobe biologisch aktive Verbindung in Phytoestrogen, ein Phytosterol, ein Isoflavon, ein Stilben, ein Lignan oder ein Coumestan; oder wobei die hydrophobe biologisch aktive Verbindung Coenzym Q10 ist.

4. Zusammensetzung, umfassend eine wieder-zusammengesetzte Kasein-Mizelle nach einem der Ansprüche 1 bis 3.

5. Zusammensetzung nach Anspruch 4, wobei die hydrophobe biologisch aktive Verbindung ein Nutraceutical ist.

6. Zusammensetzung nach Anspruch 4, wobei die Zusammensetzung ein fettarmes oder ein fettfreies Milchprodukt ist.

7. Zusammensetzung nach Anspruch 6, wobei die Zusammensetzung entrahmte Milch ist.

8. Zusammensetzung nach Anspruch 6, wobei die Zusammensetzung fettarmer oder fettfreier Joghurt ist.

9. Verfahren zur Herstellung einer wieder-zusammengesetzten Kasein-Mizelle, wobei die Mizelle mindestens eine hydrophobe biologisch aktive Verbindung in der Mizelle einschließt, wobei die biologisch aktive Verbindung ein Nutraceutical, ein Medikament oder ein kosmetisches Produkt ist, wobei das Verfahren die Schritte umfasst:
a) Herstellung einer wässrigen Lösung, umfassend eine Kaseinquelle;
b) Zugabe einer Co-Lösungsmittel-Lösung, umfassend mindestens eine Form einer hydrophoben biologisch aktiven Verbindung, zu der Kaseinlösung;
c) Zugabe einer Citrat-Ionen-Quelle, einer Phosphat-Ionen-Quelle und einer Kalzium-Ionen-Quelle zu dem Gemisch nach Schritt (b), wodurch eine Dispersion von Mizellen mit einer Größe im Nanobereich gebildet wird; und
d) Einstellung des pHs der Dispersion, wodurch die Mizellen mit einer Größe im Nanobereich stabilisiert werden.

10. Verfahren nach Anspruch 9, wobei der Schritt (c) umfasst:
(i) Zugabe der Phosphat-Ionen-Quelle und der Citrat-Ionen-Quelle zu dem Gemisch nach Schritt (b);
(ii) Herstellung einer Lösung, umfassend Kalzium-Ionen als Kalzium-Ionen-Quelle; und
(iii) Kombination des Gemisches nach Schritt (i) mit der Kalziumlösung nach Schritt (ii) unter Anwendung einer Hochdruckhomogenisierung.

11. Verfahren nach Anspruch 9 oder 10, weiterhin umfassend den Schritt der Trocknung der Mizelle-Dispersion; oder wobei die Lösung etwa 1% bis etwa 20% Kasein umfasst; oder wobei der pH auf einen pH im Bereich von etwa 6,0 bis etwa 7,5 eingestellt wird.

12. Verfahren nach Anspruch 9, wobei das Co-Lösungsmittel ein lebensmittelunbedenkliches mit Wasser mischbares organisches Lösungsmittel ist.

13. Verfahren nach Anspruch 12, wobei das Co-Lösungsmittel Ethanol ist.

14. Verfahren nach Anspruch 9, wobei die wieder-zusammengesetzten Kasein-Mizellen einen durchschnittlichen Durchmesser von 50nm bis 100nm haben.

15. Verwendung einer wieder-zusammengesetzten Kasein-Mizellen-Verbindung nach einem der Ansprüche 1 oder 9 als Nanovehikel für den Einschluss, den Schutz und die Abgabe von sensitiven hydrophoben biologisch aktiven Verbindungen, wobei die sensitiven hydrophoben biologisch aktiven Verbindungen an die hydrophoben Domänen der Kaseine adsorbiert sind.

16. Verwendung nach Anspruch 15, wobei die Kasein-Mizelle, die die sensitiven hydrophoben biologisch aktiven Verbindungen einschließt, einen durchschnittlichen Durchmesser von 50nm bis 150nm hat.

17. Verwendung nach Anspruch 15 oder 16, wobei die sensitiven hydrophoben biologisch aktiven Verbindungen Nutraceuticale, Medikamente oder kosmetische Produkte sind.

## Revendications

1. Micelle de caséines réassemblées emprisonnant au moins un composé hydrophobe exogène biologiquement actif au sein de la micelle, dans laquelle le composé hydrophobe biologiquement actif est adsorbé sur les domaines hydrophobes des caséines et est choisi dans le groupe constitué d'un médicament, d'un nutraceutique, et d'un composé cosmétique.

2. Micelle selon la revendication 1, les micelles de caséines réassemblées ayant un diamètre moyen de 50 nm à 150 nm.

3. Micelle selon la revendication 1 ou 2,
dans laquelle le composé hydrophobe biologiquement actif est choisi dans le groupe constitué d'un peptide, d'une protéine, d'un acide aminé, d'un lipide, d'un protéoglycane, d'un polysaccharide, d'une vitamine, d'une hormone, d'un médicament, d'un stéroïde, d'un agent photochimique, d'un polynucléotide, d'un aromatisant, d'un édulcorant, d'un antimicrobien et d'un conservateur ; ou
dans laquelle le composé hydrophobe biologiquement actif est une vitamine soluble dans les graisses choisie parmi la vitamine A, la vitamine E, la vitamine D et la vitamine K et des dérivés de celles-ci ; ou
dans laquelle la vitamine est la vitamine D ; ou
dans laquelle le composé hydrophobe biologiquement actif est choisi dans le groupe constitué de stérol cholestérol et de dérivés de celui-ci ; ou
dans laquelle le composé hydrophobe biologiquement actif est choisi parmi l'α-carotène, le β-carotène, le γ-carotène, le lycopène, la lutéine, la zéaxanthine, et l'astaxanthine ; ou
dans laquelle le composé hydrophobe biologiquement actif est un acide gras insaturé, de préférence où l'acide gras insaturé est choisi dans le groupe constitué d'acide linoléique, d'acide linoléique conjugué, d'acide linolénique, d'acides gras oméga-3, y compris la DHA et l'EPA et leurs esters de glycérol ; ou
dans laquelle le composé hydrophobe biologiquement actif est choisi parmi un phytooestrogène, un phytostérol, une isoflavone, un stilbène, un lignane et un coumestane ; ou
dans laquelle le composé hydrophobe biologiquement actif est le coenzyme Q10.

4. Composition comprenant une micelle de caséines réassemblées selon l'une quelconque des revendications 1 à 3.

5. Composition selon la revendication 4, dans laquelle le composé hydrophobe biologiquement actif est un nutraceutique.

6. Composition selon la revendication 4, la composition étant choisie parmi un produit laitier pauvre en graisses et sans graisse.

7. Composition selon la revendication 6, la composition étant du lait écrémé.

8. Composition selon la revendication 6, la composition étant un yaourt pauvre en graisses ou sans graisse.

9. Méthode de préparation d'une micelle de caséines réassemblées, la micelle emprisonnant au moins un composé hydrophobe biologiquement actif au sein de la micelle, où le composé biologiquement actif est choisi dans le groupe constitué d'un nutraceutique, d'un médicament et d'un produit cosmétique, la méthode comprenant les étapes suivantes :
a) la préparation d'une solution aqueuse comprenant une source de caséine ;
b) l'addition d'une solution de cosolvant comprenant au moins un type de composé hydrophobe biologiquement actif à la solution de caséine ;
c) l'addition d'une source d'ions citrate, d'une source d'ions phosphate et d'une source d'ions calcium au mélange de l'étape (b) pour former une dispersion de micelles d'une taille de l'ordre du nanomètre ; et
d) l'ajustement du pH de la dispersion pour stabiliser les micelles de la taille de l'ordre du nanomètre.

10. Méthode selon la revendication 9, dans laquelle l'étape (c) comprend :
(i) l'addition de la source d'ions phosphate et de la source d'ions citrate au mélange de l'étape (b) ;
(ii) la préparation d'une solution comprenant des ions calcium en tant que source d'ions calcium ; et
(iii) la combinaison du mélange de l'étape (i) avec la solution de calcium de l'étape (ii) en utilisant une homogénéisation à pression élevée.

11. Méthode selon la revendication 9 ou 10, comprenant en outre l'étape de séchage de la dispersion de micelles ; ou bien où la solution comprend environ 1 % à environ 20 % de caséine ; ou bien où le pH est ajusté à un pH situé dans la plage d'environ 6,0 à environ 7,5.

12. Méthode selon la revendication 9, dans laquelle le cosolvant est un solvant organique miscible à l'eau de qualité alimentaire.

13. Méthode selon la revendication 12, dans laquelle le cosolvant est l'éthanol.

14. Méthode selon la revendication 9, dans laquelle les micelles de caséines réassemblées ont un diamètre moyen de 50 nm à 100 nm.

15. Utilisation d'un composé de micelle de caséines réassemblées selon la revendication 1 ou 9, en tant que nanovéhicule pour l'emprisonnement, la protection et la délivrance de composés hydrophobes biologiquement actifs sensibles, où les composés hydrophobes biologiquement actifs sensibles sont adsorbés sur les domaines hydrophobes des caséines.

16. Utilisation selon la revendication 15, où la micelle de caséines emprisonnant les composés hydrophobes biologiquement actifs sensibles a un diamètre moyen de 50 nm à 150 nm.

17. Utilisation selon la revendication 15 ou 16, où les composés hydrophobes biologiquement actifs sensibles sont des nutraceutiques, des médicaments ou des produits cosmétiques.
